# DEMANDE DE BREVET EUROPEEN

(11) **EP 3 926 599 A2**
(43) Date de publication de la demande: **22.12.2021**
(21) Numéro de dépôt: 21180060.2
(22) Date de dépôt: 17.06.2021
(51) Int. Cl.: G08B 29/14, G08B 17/10, G08B 25/10, G08B 26/00

(54) **DISPOSITIF ET SYSTEME DE TEST POUR UN DETECTEUR BASE SUR LA DETECTION D'UN GAZ OU D'UN MELANGE GAZ/PARTICULES**

(30) Priorité: 19.06.2020 FR 2006404
(71) Demandeur: Commissariat à l'Energie Atomique et aux Energies Alternatives, 75015 Paris (FR)
(72) Inventeur: DALL'OMO, Christophe, 38054 GRENOBLE Cedex 09 (FR); SCHATZ, David, 21120 IS SUR TILLE (FR); PELISSON, Roland, 38054 GRENOBLE Cedex 09 (FR); DEPARIS, Nicolas, 38054 GRENOBLE Cedex 09 (FR); DANIELE, Norbert, 38054 GRENOBLE Cedex 09 (FR)
(74) Mandataire: Marks & Clerk France

(57) **Abrégé**

L'invention concerne un dispositif de test (1) pour un détecteur de gaz ou de mélange gaz/particules, comprenant :
- un moyen de solidarisation (11) du dispositif de test au détecteur;
- un contenant (13) configuré pour recevoir un gaz ou un mélange gaz/particules de test ;
- un moyen de diffusion (12) associé au contenant (13) et adapté pour diffuser le gaz ou le mélange gaz/particules de test dans le détecteur ;
- une source d'énergie (160) ; et
- un équipement radio (140) apte à gérer une communication radio locale pour déclencher à distance, depuis un moyen de commande, la diffusion du gaz ou du mélange gaz/particules de test dans le moyen de diffusion.

L'invention concerne également un système de test comprenant au moins un dispositif de test (1), au moins une passerelle radio, un réseau de commande comportant une unité de commande apte à commander à distance le dispositif de test et au moins une liaison de communication entre l'unité de commande et la passerelle radio, ladite passerelle étant apte à relier l'équipement radio et le réseau de commande.

## Description

### Domaine technique de l'invention

La présente invention concerne un dispositif de test pour un détecteur basé sur la détection de gaz ou de mélange gaz/particules, en particulier pour un détecteur automatique d'incendie (dit « DAI ») basé sur la détection de fumée. Le dispositif de test selon l'invention est conçu pour être solidarisé avec le détecteur, pour être autonome en énergie et pour pouvoir gérer une communication radio afin de déclencher à distance, depuis un moyen de commande, la diffusion du gaz de test ou du mélange gaz/particules de test vers le détecteur.

L'invention concerne également un système de test qui comprend un ou plusieurs dispositif(s) de test, un réseau de commande comprenant un moyen de commande et au moins une passerelle radio reliant le(s) dispositif(s) de test et l'unité de commande.

L'invention concerne également un système de détecteur comprenant un système de test et au moins un détecteur.

### Etat de la technique

La plupart des tests des détecteurs automatiques d'incendie de type détecteurs de fumée se font généralement manuellement par un opérateur et visent à amener un gaz de test et plus précisément un mélange gaz/particules de test (fumée généralement sous forme d'aérosol) au plus près de la tête de détecteur, le plus souvent à l'aide d'une perche, tout en communiquant à l'aide d'un moyen radio à disposition de l'opérateur (type radio PMR VHF/UHF) avec un système de supervision du détecteur, et ce, afin de valider ou non le test.

Il a été développé des systèmes de test plus élaborés, de type Scorpion^{®}, dans lesquels un testeur est monté à côté d'un détecteur incendie de type fumée, le testeur comprenant un rail de support qui est attaché au détecteur incendie à tester ou à la base duquel ledit détecteur incendie est monté, un corps contenant un aérosol et une buse reliée au corps à partir de laquelle un jet d'aérosol généré par le testeur est dirigé vers la tête de détection du détecteur incendie. Le testeur utilise ses propres câbles d'alimentation et de données indépendantes et il utilise un panneau de commande de test, distinct de tout câblage du système de détection incendie et du panneau de commande du système de détection incendie préinstallés. Plusieurs détecteurs d'incendie sont reliés en filaire à un panneau de commande fixe (contrôle de 1 à 8 têtes), ou à un panneau de commande portable via une borne (contrôle de 1 à 4 têtes), le tout également en filaire. Dans les deux cas, il est nécessaire de disposer d'un câble de testeur par détecteur, et en outre un câble est limité à une longueur de 100 mètres. Enfin, pour effectuer un test d'un détecteur incendie, un opérateur doit se rendre sur le site du système de détection incendie, faire passer le système en mode test, puis introduire une source d'alimentation sur le panneau de commande de test du système Scorpion^{®}, il doit ainsi être face au panneau de commande fixe ou au panneau de commande portable.

Si un tel système de test permet d'atteindre des détecteurs incendie situées en hauteur et difficilement atteignables et/ou difficilement accessibles, il est cependant nécessaire d'équiper chaque testeur en filaire. Son installation est donc relativement coûteuse du fait du déploiement nécessaire des propres câbles de commande et d'alimentation du système de test. En outre, la distance d'action est limitée à 100 mètres. De plus, le testeur nécessite la fourniture d'une quantité relativement importante d'énergie pendant le fonctionnement pour générer l'aérosol. Enfin, du fait de l'encombrement des têtes Scorpion^{®}, l'orientation du testeur affecte l'efficacité des tests effectués, le testeur pouvant uniquement s'insérer en position le long d'un certain axe (par exemple le long d'un couloir), mais le flux d'air à cet endroit peut s'opposer au passage de l'aérosol vers le composant de détection, ce qui réduit la fiabilité de tout test.

Il existe des systèmes, comme le système décrit dans la demande de brevet EP1468409A1, comprenant des détecteurs incendie, chacun étant muni d'un dispositif électronique de type RFID contenant des informations propres au détecteur (numéro d'identification, date du dernier test, résultat du test...) et un dispositif de test situé sur une perche de longueur réglable, par exemple une perche télescopique, de manière à pouvoir atteindre chacun des détecteurs et comprenant des moyens de lecture pour lire les données du dispositif électronique de type RFID. Ainsi les détecteurs et le dispositif de test peuvent communiquer par un système sans fil de proximité, mais ceci est limité à l'échange de données. Cependant, il est toujours nécessaire de disposer d'une perche pour que le dispositif de test atteigne chaque détecteur. Le test ne peut donc pas être commandé à distance. La demande de brevet EP2988282A1 décrit également un détecteur incendie dans laquelle est installée une puce RFID qui permet la communication et le transfert de données entre le détecteur incendie et un outil de test du détecteur cette fois-ci sans perche, mais ceci est également limité à l'échange de données et ce, à proximité. Le test ne peut donc pas être commandé à distance.

Il existe des systèmes de détection incendie qui intègrent des moyens de test, dits « systèmes d'auto-test », comme décrit dans la demande US2015302727A1 qui indique que les systèmes d'auto-test ne permettent pas toujours de valider complètement le fonctionnement des détecteurs incendie, notamment lorsqu'une voie d'un détecteur d'incendie est obstruée et qui propose un dispositif pour déterminer si une voie est obstruée. Le dispositif proposé comprend une source de lumière pour diriger la lumière dans les voies et un ou plusieurs capteurs positionnés dans le détecteur de manière à pouvoir analyser la lumière qui est rentrée. Le dispositif permet ainsi de détecter les obstructions dans les voies en analysant la façon dont la lumière se propage à travers les voies. Dans le système de détection incendie décrit, chaque détecteur incendie comprend dans une unité de base des composants de communication permettant au détecteur de communiquer avec un panneau de commande via une liaison filaire (réseau de communication de sécurité). La demande de brevet GB2543065A décrit ce problème d'obstruction, mais répond plutôt aux inconvénients des systèmes de type Scorpion^{®} décrits précédemment. Elle décrit un dispositif de test de détecteur incendie comprenant un réservoir de liquide et un générateur d'aérosol du type à mailles vibrantes en connexion fluidique avec le réservoir de liquide pour générer un aérosol à partir du réservoir de liquide, agencé de telle sorte que, lorsqu'il est généré, l'aérosol est dirigé vers l'élément détecteur d'un détecteur d'incendie.

Dans ces deux dernières demandes de brevet, la fonction d'auto-test est pilotée par le réseau de communication de sécurité des détecteurs incendie, et elle est incorporée dans les détecteurs incendie, ce qui nécessite de changer tous les détecteurs incendie pour obtenir cette nouvelle fonction. Ce n'est donc pas une fonction que l'on vient ajouter aisément sur des détecteurs incendie.

L'invention vise à surmonter les inconvénients précités de l'art antérieur.

Plus particulièrement elle vise à disposer d'un dispositif de test universel pour détecteur incendie et plus généralement pour tout détecteur basé sur la détection de gaz ou de mélange gaz/particules, qui puisse être aisément adaptable à n'importe quel détecteur existant et/ou déjà installé, qui soit contrôlable à distance (notamment à plus de 100 mètres), ce qui est notamment recherché pour des détecteurs situés en hauteur, difficilement atteignables et/ou difficilement accessibles.

Il est recherché en outre un dispositif de test autonome, qui permette de s'affranchir de perches, de nacelles ou autres moyens pour s'approcher du détecteur à tester, et qui permette de s'affranchir de quantité et de longueurs importantes de câbles.

Il est recherché en outre un système de test qui soit de faible facture énergétique, qui soit rapide à mettre en œuvre, et qui soit fiable.

Il est également recherché un test qui soit simple à mettre en œuvre, et puisse notamment être mis en œuvre sans opérateur.

### Exposé de l'invention

Un dispositif permettant de remédier à ces inconvénients est un dispositif de test pour un détecteur basé sur la détection de gaz ou de mélange gaz/particules, ledit dispositif comprenant :
- un moyen de solidarisation adapté pour solidariser le dispositif de test au détecteur à tester ;
- un contenant configuré pour recevoir un gaz de test ou un mélange gaz/particules de test ;
- un moyen de diffusion associé au contenant et adapté pour diffuser le gaz de test ou le mélange gaz/particules de test dans le détecteur ;
- une source d'énergie, par exemple un moyen de stockage d'énergie, adaptée pour rendre le dispositif de test autonome en énergie et
- un équipement radio apte à gérer une communication radio locale, de préférence une communication radio bidirectionnelle, pour au moins déclencher à distance, depuis un moyen de commande, la diffusion du gaz test ou du mélange gaz/particules de test dans le moyen de diffusion, et ainsi dans le détecteur.

La communication radio peut être établie avec un réseau de commande comprenant un moyen de commande, et ce, par l'intermédiaire d'au moins une passerelle radio.

Le dispositif de test selon l'invention peut en outre comporter l'un ou plusieurs des modes de réalisation suivants pris isolément ou suivant toutes combinaisons techniquement possibles.

Selon un mode de réalisation, le contenant est apte à recevoir ou est une cartouche de gaz test ou de mélange gaz particules test.

Selon un mode de réalisation, le moyen de diffusion comprend un diffuseur configuré pour acheminer le gaz de test ou le mélange gaz/particules de test au plus près de l'entrée de gaz du détecteur.

Selon un mode de réalisation, le moyen de diffusion comprend un élément apte à coopérer avec le moyen de solidarisation.

Selon un mode de réalisation, le dispositif de test comprend en outre un actionneur pilotable à distance et apte à actionner la diffusion du gaz de test ou du mélange gaz/particules de test dans le moyen de diffusion, et ainsi dans le détecteur.

Selon un mode de réalisation, le moyen de solidarisation comprend au moins un doigt d'accrochage dont une première extrémité présente une forme en crochet apte à coopérer avec un renfoncement dans le détecteur.

Selon un mode de réalisation, le moyen de solidarisation comprend une bride de fixation apte à enserrer le détecteur.

Selon un mode de réalisation particulier, le moyen de solidarisation comprend un moyen de réglage de la position relative du dispositif de test par rapport au détecteur.

Selon un mode de réalisation particulier, le moyen de solidarisation comprend en outre au moins une extrémité adaptée pour régler le serrage ou le desserrage de la bride de de fixation autour du détecteur, ladite au moins une extrémité étant de préférence positionnée dans l'axe du centre de gravité du dispositif de test.

De préférence, la communication radio locale est conforme à la technologie LoRa, et l'équipement radio comprend un composant compatible avec la technologie LoRa, avec un protocole de communication basé sur le protocole LoRaWAN.

De préférence, le dispositif de test comprend une base de temps précise, par exemple une horloge en temps réel (RTC pour « Real Time Clock » en anglais).

En outre, l'équipement radio comprend de préférence un moyen de mise en veille et de réveil périodique, la périodicité de réveil étant adaptable. La durée de veille peut également être adaptable.

Selon un mode de réalisation particulier, le moyen de mise en veille et de réveil périodique du dispositif de test est intégré dans une couche logicielle adossée sur la couche LoRaWAN de l'équipement radio du dispositif de test.

L'invention concerne également un système de test comprenant au moins un dispositif de test selon l'invention, et comprenant en outre :
- au moins une passerelle radio, et
- un réseau de commande comportant une unité de commande apte à commander à distance le au moins un dispositif de test et au moins une liaison de communication réseau entre ladite unité de commande et ladite au moins une passerelle radio ;
la au moins une passerelle radio étant apte à relier l'équipement radio d'au moins un dispositif de test et le réseau de commande.

Par passerelle radio (« gateway » en anglais), on entend un dispositif permettant de relier deux réseaux de communication de types différents. Selon l'invention, la passerelle radio permet de relier l'équipement radio et le réseau de commande comprenant le moyen de commande. Dans la présente description, le terme « passerelle » désigne par raccourci une passerelle radio.

Un équipement radio contribue à former un réseau radio local à l'aide d'une ou de plusieurs passerelles radio. Lorsqu'il y a plusieurs dispositifs de test, il y a plusieurs équipements radio dans le réseau radio local. Ainsi le réseau radio local désigne le réseau formé par la (ou les) dispositif(s) de test, et la (ou les) passerelle(s).

L'équipement radio permet d'échanger des informations avec le réseau de commande via une passerelle radio, de préférence de manière bidirectionnelle.

Le réseau de commande désigne le réseau formé par la (ou les) passerelle(s) et l'unité de commande, avec la liaison de communication les reliant, et peut comprendre d'autres éléments insérés entre la (ou les) passerelle(s) et l'unité de commande.

Le réseau de commande peut comprendre ou consister en un réseau informatique IP. On rappelle qu'un réseau informatique IP est un réseau de communication sous protocole Internet (ou « IP » pour « Internet Protocol » en anglais). Dans la présente description, le terme « réseau IP » ou « réseau informatique IP » désigne par raccourci un réseau informatique sous protocole Internet. De même une « liaison IP » ou « liaison de communication IP » ou « liaison de communication sous IP » désigne une liaison de communication sous protocole Internet.

De préférence, le réseau de commande comprend un serveur de diffusion de temps universel apte se synchroniser avec une base de temps dans le dispositif de test, de manière à disposer d'une base de temps commune.

De préférence, l'unité de commande est apte à donner des instructions de mise en veille et de réveil au dispositif de test, la périodicité de réveil étant adaptable. La durée de veille est également adaptable. Cela permet d'améliorer l'autonomie du dispositif de test.

Le réseau de commande peut être un réseau IP, l'unité de commande comprenant alors une couche IP, l'unité de commande étant par exemple un ordinateur de bureau, un ordinateur portable, une tablette, un téléphone intelligent...

Selon un mode de réalisation, la au moins une liaison de communication réseau est entièrement filaire.

Selon un mode de réalisation, le réseau de commande comprend un concentrateur réseau et une interface de communication ayant un protocole de communication compatible avec l'équipement radio et la au moins une passerelle radio, généralement un concentrateur applicatif.

De préférence, la communication radio locale est conforme à la technologie LoRa, l'équipement radio du dispositif de test comprend un composant compatible avec la technologie LoRa, avec un protocole de communication basé sur le protocole LoRaWAN et la au moins une passerelle radio est une passerelle LoRaWAN.

Selon un mode de réalisation particulier, le réseau de commande comprend un concentrateur réseau et un concentrateur applicatif.

Selon un mode de réalisation particulier, le moyen de mise en veille et de réveil périodique du dispositif de test est intégré dans une couche logicielle adossée sur la couche LoRaWAN de l'équipement radio du dispositif de test et sur le concentrateur applicatif.

Selon un mode de réalisation, le réseau de commande comprend plusieurs liaisons de communication comportant une partie filaire reliant la au moins une passerelle, le concentrateur réseau et le concentrateur applicatif, et une partie sans-fil, par exemple en Wifi, reliant le concentrateur applicatif et l'unité de commande. Le réseau de commande peut être un réseau IP, et les liaisons de communication réseau peuvent être des liaisons de communication IP.

Selon un mode de réalisation, le réseau de commande comprend plusieurs liaisons de communication comportant au moins une liaison de communication IP, filaire et/ou sans-fil, entre la au moins une passerelle, le concentrateur réseau et le concentrateur applicatif et au moins une liaison de communication radio LoRa entre la au moins une passerelle et l'unité de commande, l'unité de commande comprenant un dispositif portable équipé d'un composant radio compatible avec la technologie LoRa.

Le dispositif de test et le système de test selon l'invention peuvent comporter l'une quelconque des caractéristiques précédemment énoncées, prises isolément ou selon toutes combinaisons techniquement possibles avec d'autres caractéristiques.

L'unité de commande peut être comprise dans ou être associée avec une unité de supervision d'un ou de plusieurs détecteurs.

L'invention concerne aussi un système de détection comprenant un système de test selon l'invention et un détecteur basé sur la détection de gaz ou de mélange gaz/particules.

Selon un mode de réalisation préféré, le détecteur est un détecteur incendie basé sur la détection de fumée.

### Brève description des figures

D'autres caractéristiques et avantages de l'invention apparaîtront à l'aide de la description qui suit, donnée à titre illustratif et non limitatif, faite en regard des figures annexées parmi lesquelles
[Fig.1A] et
[Fig. 1B] représentent un premier mode de réalisation de dispositif de test conforme à l'invention.
[Fig. 2A] représente une première variante de dispositif de test conforme à l'invention.
[Fig. 2B] représente une deuxième variante de dispositif de test conforme à l'invention.
[Fig. 3] représente une troisième variante de dispositif de test conforme à l'invention.
[Fig. 4A] et
[Fig. 4B] représentent une quatrième variante de dispositif de test conforme à l'invention.
[Fig. 5] représente un système de test conforme à l'invention.
[Fig. 6A] et
[Fig. 6B] représentent un premier mode de réalisation de système de test conforme à l'invention [Fig. 7A] et
[Fig. 7B] représentent un deuxième mode de réalisation de système de test conforme à l'invention.
[Fig. 8A] et
[Fig. 8B] représentent un troisième mode de réalisation de système de test conforme à l'invention
[Fig. 9] représente un graphe d'état illustrant un exemple de protocole de fonctionnement d'un système de test.

Dans l'ensemble de ces figures, des références identiques peuvent désigner des éléments identiques ou analogues.

De plus, les différentes parties représentées sur les figures ne le sont pas nécessairement selon une échelle uniforme, pour rendre les figures plus lisibles.

### Description détaillée de l'invention

Dans la suite de la description, le terme « gaz test » est utilisé pour décrire indifféremment : un gaz, un mélange de différents gaz ou encore un mélange de gaz et de particules, adapté pour tester un détecteur basé sur la détection de gaz ou de mélange gaz/particules.

En outre, il est précisé que lorsqu'un élément est décrit comme étant relié, connecté, assemblé, solidarisé à un autre élément, et sauf indication contraire, il peut être directement relié, connecté, assemblé, solidarisé à l'autre élément ou des éléments intermédiaires peuvent être présents.

En outre, le dispositif de test est décrit dans la description détaillée en référence à un détecteur à incendie de type fumée, qui peut être nommé « détecteur de fumée », bien qu'il puisse s'appliquer à tout autre détecteur basé sur la détection de gaz ou de mélange gaz/particules, dans la portée des revendications. Il en est de même pour le système de test et le système de détection.

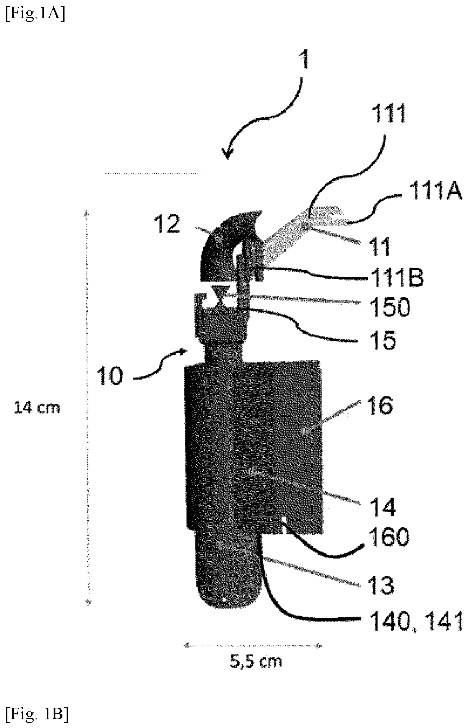

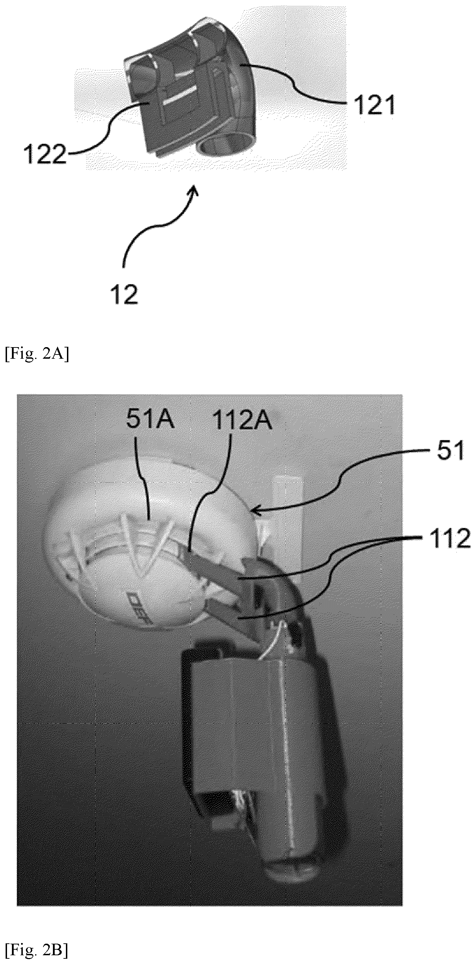

Les figures 1A et 1B illustrent un premier mode de réalisation de dispositif de test conforme à l'invention qui se présente sous la forme d'un corps principal 10 relié à un moyen de solidarisation 11.

Le corps principal peut être en plastique, en métal ou tout autre matériau adapté, de préférence léger et résistant aux chocs et dans le temps. La hauteur du corps principal du dispositif de test illustré est de 14 cm, sa profondeur est d'environ 4 cm et sa largeur est d'environ 5,5 cm.

Le moyen de solidarisation illustré en figure 1A comprend au moins un doigt d'accrochage 111 dont une première extrémité 111A présente une forme en crochet apte à coopérer avec un renfoncement dans un détecteur (non représenté dans les figures 1A et 1B).

Un détecteur de fumée comprend des ouvertures d'entrée de gaz, par exemple sous forme d'ailettes, par lesquelles la fumée peut s'introduire à l'intérieur du détecteur pour atteindre les composants de détection. Ces ouvertures peuvent former un ou plusieurs renfoncements dans le(s)quel(s) un doigt d'accrochage peut s'insérer.

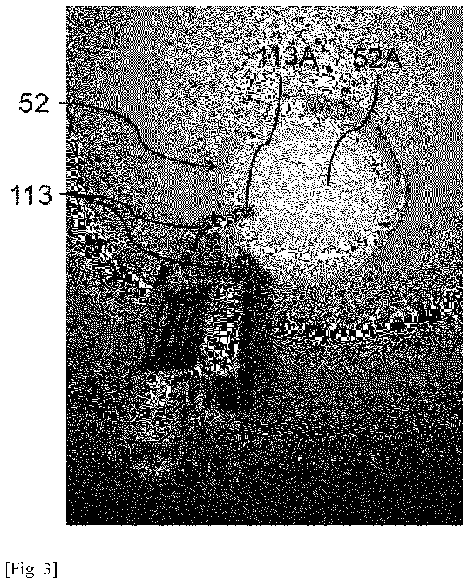

Les figures 2A et 2B illustrent deux variantes de moyens de solidarisation sous forme de doigts d'accrochage avec deux types de doigts différents permettant de s'adapter à deux types de détecteurs différents.

Dans une première variante, en figure 2A, sont représentés deux doigts 112 dont les premières extrémités 112A sont adaptées pour s'insérer verticalement entre deux ailettes verticales 51A d'un premier type de détecteur 51.

Dans une deuxième variante, en figure 2B, sont représentés deux doigts 113 dont les premières extrémités 113A sont adaptées pour s'insérer horizontalement entre deux ailettes horizontales 52A d'un deuxième type de détecteur 52.

Cela permet dans les deux variantes, de garantir une direction optimale de diffusion du gaz test dans les ouvertures du détecteur.

Les moyens de solidarisation sous forme de doigts d'accrochage sont simples à mettre en œuvre et peuvent être aisément adaptés à des détecteurs différents, par exemple en adaptant la forme du doigt (ou des doigts) et notamment en adaptant la forme de l'extrémité du doigt (ou des doigts) destinée à coopérer avec le détecteur. Les formes décrites ne sont pas limitatives et l'homme du métier saura les adapter à d'autres types de détecteurs.

Toutefois, il existe des détecteurs qui ne comprennent pas d'ouvertures aisées à crocheter avec un ou plusieurs doigt(s) d'accrochage, ou des détecteurs pour lesquelles une solidarisation avec des doigts ne serait pas sûre dans la durée. En outre, les doigts peuvent présenter des zones de fragilité, et peuvent finir par casser au bout de plusieurs accrochages et décrochages. Enfin, un tel moyen d'accrochage peut parfois requérir une trop grande précision pour accrocher le détecteur, ce qui n'est pas toujours évident à réaliser en hauteur.

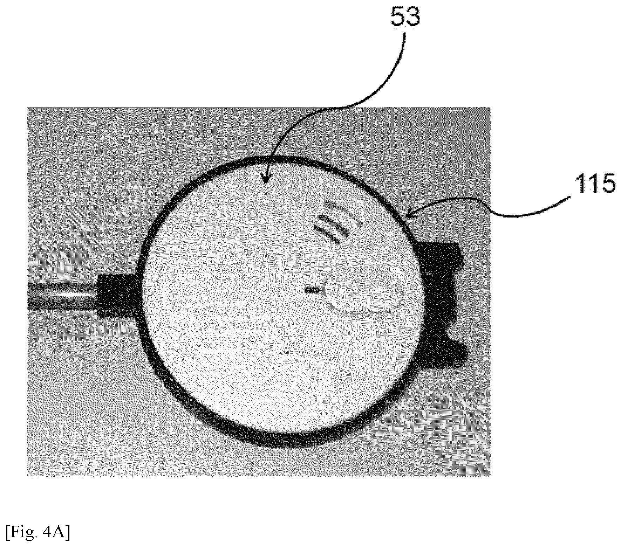

La figure 3 illustre une troisième variante dans laquelle un troisième type de détecteur 53 est représenté, qui ne comprend pas d'ailettes ou d'ouvertures aisées à crocheter. Le moyen de solidarisation 115 illustré se présente sous la forme d'une bride de fixation de type collier de serrage qui vient encercler et enserrer le détecteur 53. Une telle bride de fixation convient ici à tout type de détecteur de forme générale circulaire, mais d'autres brides peuvent être adaptées pour des formes non complètement circulaires. La première bride de fixation peut être en matière plastique, en matière métallique, ou mixte.

Le moyen de solidarisation décrit en référence avec la figure 3 est illustré avec le troisième type de détecteur 53, mais il peut être adapté à tout type de détecteur. Les autres éléments du dispositif de test peuvent être identiques à ceux décrits plus après dans la présente description.

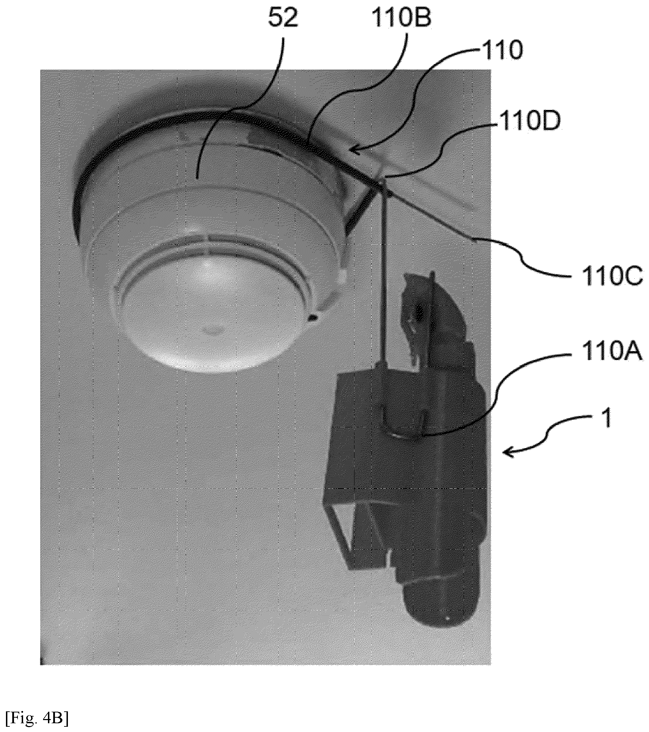

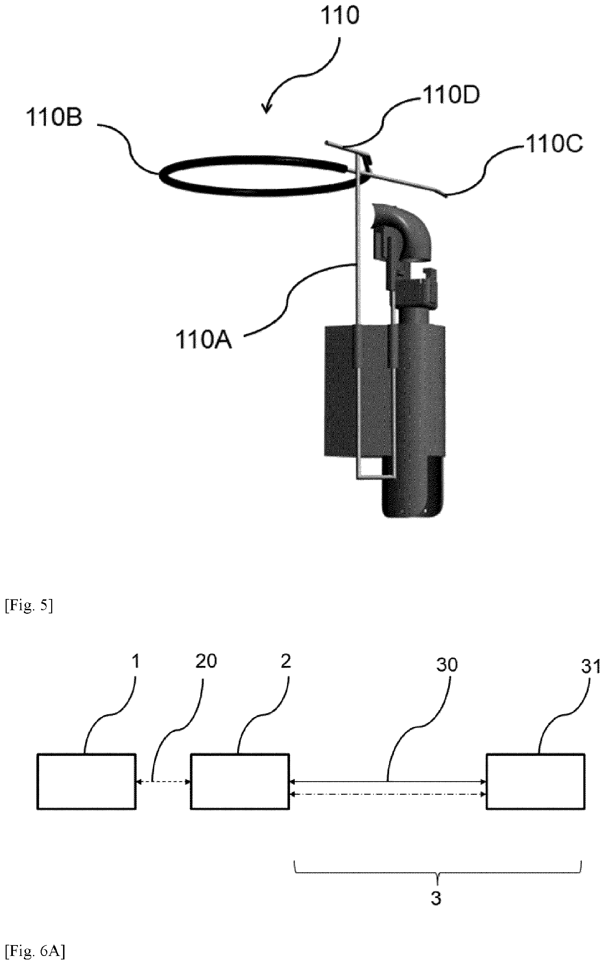

Les figures 4A et 4B illustrent une quatrième variante dans laquelle le moyen de solidarisation 110 se présente sous la forme d'un fil métallique présentant des propriétés de ressort (ou de rappel), ledit fil métallique étant conformé pour permettre les fonctions suivantes :
- le positionnement du dispositif de test sur le fil métallique à une hauteur définie (de préférence avant son assemblage avec le détecteur), grâce à une première partie 110A du fil métallique formant une boucle en U : la boucle en U coopère avec le corps principal du dispositif de test de manière à autoriser la translation dudit dispositif de test jusqu'à une position souhaitée du moyen de diffusion vis-à-vis du détecteur ;
- la fixation du dispositif de test autour du détecteur grâce à une seconde partie 110B du fil formant une bride de fixation (ou collier de serrage), ladite bride de fixation étant dimensionnée pour enserrer le détecteur : la force de serrage de la bride de fixation appliquée autour du détecteur doit être suffisante pour le serrer tout en supportant le poids du dispositif de test, en évitant que celui-ci ne glisse ; de préférence, la partie du fil métallique formant bride de fixation est entourée d'un matériau tampon antidérapant, tel un caoutchouc, permettant le maintien du dispositif de test autour du détecteur (et évitant le glissement) ;
- la préhension de la bride de fixation 110B, pour sa mise en place et son retrait, via une première extrémité 110C du fil métallique et une deuxième extrémité 110D du fil métallique pouvant facilement être saisies, par exemple via une pince télescopique munie de tampons ou de ventouses souples.

En effectuant une pression sensiblement horizontale sur les bouts des deux extrémités, il est possible d'augmenter le diamètre de la bride et notamment de desserrer le détecteur. En relâchant la pression sur les deux extrémités, il est possible de diminuer le diamètre de la bride et notamment de serrer le détecteur.

Les extrémités 110C et 110D sont avantageusement positionnées dans l'axe du centre de gravité du dispositif de test. De cette manière, le dispositif de test se positionne naturellement verticalement tout au long du processus de saisie par les ventouses jusqu'à sa pose autour du détecteur. La pose est ainsi grandement facilitée.

La première partie 110A du fil métallique formant système de réglage en hauteur est reliée à la seconde partie 110B du fil métallique par soudure. Alternativement la première partie 110A du fil métallique peut être reliée à la seconde partie 110B via la deuxième extrémité 110D en réalisant un pliage du fil métallique au niveau de ladite deuxième extrémité (un seul fil métallique pour former le tout).

Cette bride de fixation est dite « universelle » (en ce sens qu'elle n'est pas conçue pour un type unique de détecteur) et elle est avantageuse en ce qu'elle permet le réglage du dispositif de test relativement au détecteur. Un réglage en hauteur du dispositif de test permet de s'assurer que le diffuseur est bien disposé en face des ouvertures d'entrée de gaz du détecteur, par exemple en face des ailettes lorsque le détecteur en est équipé. Elle est aussi avantageuse par sa facilité de mise en place sur le détecteur (notamment elle s'aligne automatiquement autour de ce dernier par gravité). Elle est également avantageuse car peut être facilement déposée : en effet, les ventouses ou les tampons n'ont pas besoin d'être parfaitement alignés pour la saisir.

Le moyen de solidarisation décrit en référence avec les figures 4A et 4B est illustré avec le deuxième type de détecteur 52, mais il peut être adapté à tout type de détecteur. Les autres éléments du dispositif de test peuvent être identiques à ceux décrits plus après dans la présente description.

L'extrémité supérieure du corps principal 10 est ouverte et elle est profilée pour former un moyen de diffusion 12 comprenant un diffuseur 121 dimensionné pour acheminer le gaz test au plus près de la tête du détecteur.

Il est précisé que la tête du détecteur désigne la partie de détecteur qui comprend les composants de détection.

Le moyen de diffusion 12 détaillé en figure 1B comprend une première partie 121 formant le diffuseur qui présente une forme de conduit coudé relié au contenant 13 de gaz test et dimensionné pour acheminer le gaz test au niveau des ouvertures du détecteur, de sorte que le gaz test puisse atteindre la tête du détecteur. Le conduit courbé assure une diffusion du gaz test par un effet venturi en entraînant le gaz test dans les ailettes du détecteur après une déviation à 90°.

Il est précisé qu'un diffuseur est défini comme un organe statique permettant de répartir un flux gazeux.

Le moyen de diffusion 12 illustré en figure 1B comprend en outre une seconde partie 122 profilée pour coopérer avec les moyens de solidarisation, par exemple avec la seconde extrémité 111B du doigt 111, comme illustré en figure 1A.

Le moyen de solidarisation peut être maintenu à demeure sur le détecteur et le reste du dispositif de test peut être solidarisé ou désolidarisé avec ledit moyen de solidarisation, par exemple via cette seconde partie 122 du moyen de diffusion 12, en déplaçant le moyen de diffusion relativement au moyen de solidarisation, par exemple en le faisant coulisser de façon à récupérer et à repositionner facilement le moyen de diffusion, par exemple lors de la maintenance du dispositif de test (pour recharger la cartouche de gaz test notamment).

Dans le cas où le moyen de solidarisation est maintenu sur le détecteur, le moyen de solidarisation comprend de préférence au moins trois points de fixation avec le détecteur (par exemple trois doigts ou une bride de fixation, ou encore tout autre moyen adapté) pour empêcher que le diffuseur ne se détache en cas de vibration ou choc (en cas de séisme par exemple).

Un premier compartiment du corps principal forme un contenant 13 dimensionné pour recevoir une cartouche de gaz contenant le gaz test, la cartouche étant par exemple de type standard et pouvant comprendre une valve située en partie supérieure de la cartouche. Alternativement, le contenant peut être la cartouche de gaz, et le dispositif de test comprend un moyen pour maintenir ladite cartouche.

A titre d'illustration, on peut utiliser une cartouche standard en aluminium, de capacité 24 millilitres, de diamètre 25 millimètres et de longueur 90 millimètres. La cartouche peut être rechargeable ou à usage unique.

La valve de la cartouche peut être :
- une valve continue (ou à action verticale) : le gaz test est libéré tant qu'une pression est formée sur la valve ;
- une valve doseuse : le gaz test peut être libéré suivant un volume bien précis ;
- une valve à usage unique qui libère tout le gaz test de la cartouche (opercule percé lors de la mise en place).

Pour un détecteur de fumée, le type de gaz employé est de la fumée pour testeur de détecteur de fumée (« SDT » pour Smoke Detector Test en anglais), sous forme d'aérosol.

Dans le dispositif de test illustré en figure 1, un deuxième compartiment 16 du corps principal est adapté pour recevoir une source d'énergie 160 qui peut être un moyen de stockage d'énergie (par exemple une ou plusieurs piles ou une batterie rechargeable ou tout autre moyen adapté).

En outre, le dispositif de test illustré en figure 1 comprend un actionneur 150 apte à actionner l'introduction du gaz test de la cartouche de gaz dans le diffuseur (et ainsi à le diffuser dans le détecteur). L'actionneur peut être une électrovanne ou un actionneur mécanique appuyant sur la valve de la cartouche.

L'actionneur relie fluidiquement le diffuseur et la cartouche de gaz test. Il peut être disposé dans un troisième compartiment 15 du corps principal situé entre le moyen de diffusion et le contenant de gaz test. L'actionneur 150 est connecté à la source d'énergie 160 pour être alimenté et à une carte électronique 141 pour être piloté. La carte électronique est de préférence dédiée à l'actionneur et doit permettre d'assurer un courant d'appel suffisant à son bon fonctionnement.

L'actionneur 150 peut être une électrovanne. Le choix d'une électrovanne est préféré. En effet, actionner l'introduction du gaz test par action mécanique sur la valve de la cartouche nécessite un effort important pour comprimer le ressort de la valve (et en outre, cela peut manquer de précision au niveau de la quantité de gaz émise, selon le type de valve disponible sur la cartouche).

Une électrovanne peut comprendre sur une de ses extrémités une emboiture femelle munie d'un joint torique d'étanchéité et conçue pour se raccorder sur la partie mâle de la cartouche de gaz, et sur son autre extrémité un opercule pour atomiser le gaz (par exemple trou fin dans une plaque métallique).

Dans le dispositif de test illustré en figure 1A, l'équipement radio 140 est disposé dans un quatrième compartiment 14 du corps principal formant partie électronique et dimensionné pour recevoir des équipements électroniques. Le quatrième compartiment peut également recevoir la carte électronique 141 de l'actionneur 150.

De préférence, le réseau radio local entre un dispositif de test et une passerelle est conforme au protocole radio LoRaWAN. LoRaWAN est l'acronyme de « Long Range Wide-Area Network » en anglais que l'on peut traduire par « réseau étendu à longue portée ». Le LoRaWAN permet des communications longues portées à bas coût et à basse consommation. Cela peut être un protocole de communication LoRaWAN bas débit (0,3 à 50 kbps).

L'équipement radio 140 peut alors comprendre une carte électronique fonctionnant sur la technologie radio LoRa, fonctionnant de préférence autour de la fréquence de 868 MHz (pour l'Europe), 915 MHz (pour les USA), ou 780 MHz (pour l'Asie), cette fréquence étant un compromis entre couverture radio et capacité radio. LoRa est la technologie de modulation liée à LoRaWAN. La carte électronique peut être une carte de développement LoRa de type Internet des Objets (« IoT »), par exemple une carte électronique LoPy4 du fabricant Pycom, disposant d'un module radio LoRaWAN, ou encore une carte radio intégrant une puce LoRa et développée spécifiquement pour les applications visées par l'invention de manière à gagner en efficacité, en consommation, en encombrement et en coût de fabrication. Une antenne radio est connectée sur la carte de développement LoRa via un connecteur ou est imprimée directement sur la carte électronique contenant le module radio de manière à s'affranchir du câble et à gagner en encombrement et en coût.

De manière plus générale, l'équipement radio 140 peut comprendre tout composant compatible avec la technologie LoRa.

La partie électronique 14 peut également comprendre une base de temps précise à très faible consommation énergétique. Cette base de temps peut être par exemple une horloge en temps réel (ou « RTC » pour Real Time Clock en anglais).

Ainsi, le dispositif de test peut être aisément attaché à un détecteur (grâce à un (ou des) doigt(s) d'accrochage, à une bride de fixation, ou grâce à tout autre moyen adapté). Le dispositif de test est rendu autonome puisqu'il intègre une source d'énergie, une cartouche de gaz test. Le dispositif de test comprend de préférence un actionneur pour actionner la sortie du gaz test depuis la cartouche vers le diffuseur, l'actionneur étant alimenté par la source d'énergie, ainsi qu'un dispositif électronique, telle qu'une carte électronique, pour piloter l'actionneur. Enfin, le dispositif de test comprend un équipement radio, tel un module radio LoRa, qui lui permet de communiquer à distance avec une unité de commande disposée dans un réseau de commande via une passerelle radio (apte à relier le réseau radio local et le réseau de commande, de préférence de manière bidirectionnelle). Cette communication est de préférence réalisée à des moments horodatés dont la référence de temps est assurée par l'utilisation d'une base de temps précise à faible consommation énergétique.

Le dispositif de test peut ainsi actionner sur commande à distance, via un protocole radio, une certaine quantité paramétrable de gaz test qui vient diffuser au niveau des ouvertures d'entrée de gaz d'un détecteur de façon à déclencher ledit détecteur (dont l'alarme peut être préalablement inhibée pour réaliser le test), et à en tester le fonctionnement à distance.

La figure 5 illustre un système de test conforme à l'invention, comprenant un dispositif de test 1, une passerelle radio 2 et un réseau de commande 3.

Une liaison de communication radio 20 (représentée par une double flèche en pointillés) relie sans fil le dispositif de test 1 et la passerelle 2. De préférence, la communication entre le dispositif de test et la passerelle se fait de manière bidirectionnelle.

Le réseau de commande 3 comprend au moins une unité de commande 31 et une liaison de communication réseau 30 entre l'unité de commande 31 et la passerelle radio 2. De préférence, la communication entre la passerelle et le réseau de commande se fait de manière bidirectionnelle.

La liaison de communication réseau 30 peut être entièrement filaire (représenté par une double flèche en trait plein).

Alternativement, la liaison de communication réseau 30 peut être sans fil (alternative représentée par une double flèche en trait mixte). Par exemple la passerelle peut utiliser une borne WIFI ou une station cellulaire déjà existante et communiquer ainsi sans fil avec l'unité de commande.

Alternativement, avec une unité de commande radio, la passerelle peut communiquer avec ladite telle unité de commande selon un protocole radio, par exemple le même protocole radio que pour la communication radio entre le dispositif de test et la passerelle. Dans ce cas, l'unité de commande radio est adaptée pour ce protocole radio. Cela peut-être une télécommande avec un composant radio adapté au protocole radio. Une telle télécommande radio peut être disposée de manière à commander le dispositif de test à plus courte distance, par exemple dans la pièce ou dans le bâtiment où se situe le détecteur. Par exemple, si l'unité de commande radio est une télécommande LoRaWAN, il est important de pouvoir se rapprocher du dispositif de test afin d'observer le comportement du détecteur lorsqu'on déclenche le test par la télécommande.

Encore alternativement, la liaison de communication réseau peut être mixte, c'est-à-dire avec une partie sans fil et une partie en filaire.

Le système de test peut comprendre plusieurs dispositifs de test associés à autant de détecteurs, et également plusieurs passerelles radio, et un réseau de commande avec une unité de commande, le réseau de commande étant connecté aux passerelles par des liaisons de communication filaires, sans-fil ou mixtes. Une passerelle peut servir de relai de communication pour plusieurs dispositifs de test, par exemple si les dispositifs de test sont situés dans la zone de couverture radio de la passerelle.

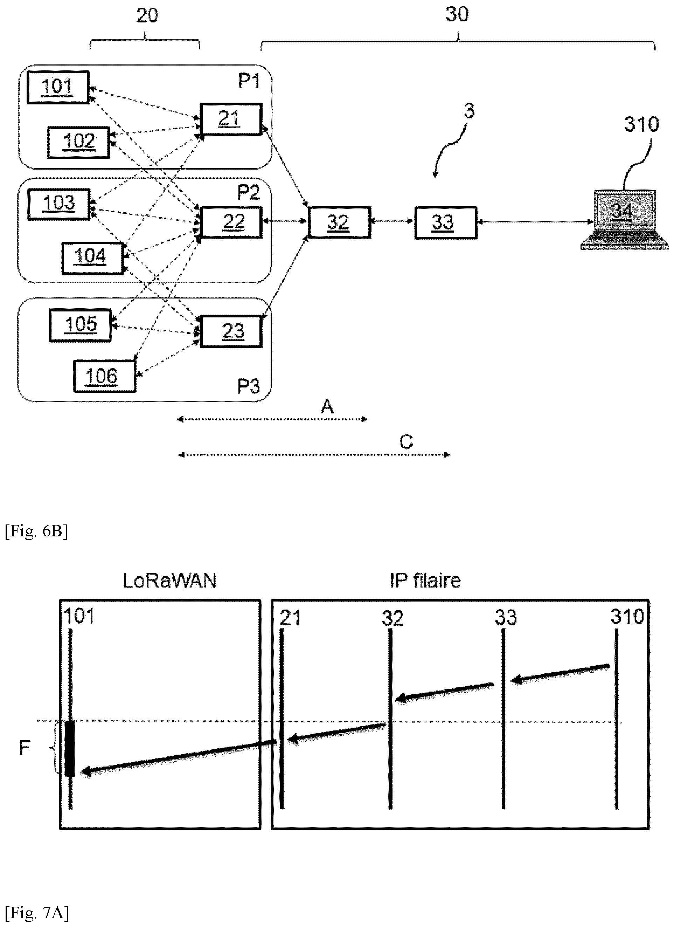

Les figures 6A-6B, 7A-7B et 8A-8B illustrent des deuxième, troisième et quatrième modes de réalisation d'un système de test présentant une architecture réseau en étoile, comprenant plusieurs dispositifs de test 101, 102, 103, 104, 105, 106 chacun étant muni d'un équipement radio, et plusieurs passerelles 21, 22, 23. Les dispositifs de test et les passerelles sont répartis dans trois zones différentes (trois pièces P1, P2 et P3 différentes). Le système de test comprend un réseau 3 relié à chacune des passerelles.

Comme pour la figure 5, les liaisons de communication radio 20 sont représentées par des doubles flèches en pointillés.

En outre, une liaison de communication réseau 30 est représentée par une double flèche en trait plein (filaire) ou une double flèche en trait mixte (sans-fil ou mixte) ou encore par une double flèche en pointillés, s'il s'agit du même protocole radio que pour la communication radio entre le(s) dispositif(s) de test et la (les) passerelle(s).

De préférence, les équipements radio des dispositifs de test communiquent de manière bidirectionnelle avec les passerelles radio. De même, les passerelles communiquent de préférence de manière bidirectionnelle avec le réseau de commande. La bidirectionnalité est avantageuse car elle permet dans un sens qu'un dispositif de test reçoive la commande d'actionnement et dans l'autre sens que le dispositif de test accuse réception de la commande, confirme que le test a été réalisé (par exemple si le test ne déclenche pas le détecteur) et/ou renseigne sur le niveau de la batterie et/ou de la cartouche de gaz ...

De préférence, chaque dispositif de test et chaque passerelle possède un identifiant unique de façon à améliorer la sécurité du système de test, notamment pour authentifier les équipements radio. Un mécanisme de chiffrement peut également être assuré à l'aide d'un mécanisme de partage de clefs numériques.

Selon les architectures illustrées en figures 6A, 7A et 8A, la passerelle 21 peut servir de relai de communication pour les dispositifs de test 101 et 102 de la même pièce P1, mais aussi de la pièce adjacente P2. La passerelle 23 peut servir de relai de communication pour les dispositifs de test 105 et 106 de la même pièce P3, mais aussi de la pièce adjacente P2. La passerelle 22 qui est dans la pièce intermédiaire P2 peut servir de relai de communication pour les six dispositifs de test qui sont dans les trois pièces.

Chaque passerelle est reliée au réseau de commande par une liaison filaire. Alternativement une passerelle peut être reliée au réseau de commande par une liaison sans fil de type WIFI ou cellulaire, ou encore par une liaison mixte.

Dans les systèmes illustrés en figures 6A-6B, 7A-7B et 8A-8B, le réseau radio local entre les dispositifs de test et les passerelles est conforme à la technologie radio LoRa, autour de la fréquence de 868 MHz, avec un protocole de communication LoRaWAN.

L'équipement radio de chaque dispositif de test comprend au moins un module radio LoRaWAN intégrant une antenne radio de manière à établir une liaison radio LoRa avec une ou plusieurs passerelles, et les passerelles sont des passerelles LoRaWAN.

Des paramètres de configuration (parmi d'autres) de la technologie LoRa sont le facteur d'étalement de spectre (« Spreading Factor » ou « SF » en anglais), la largeur de bande (« Bandwidth » ou « BW » en anglais) et le codage canal (« Coding Rate » ou « CR »). Plus particulièrement dans une architecture LoRaWAN, à chacun de ces facteurs d'étalement de spectre SF, dans une largeur de bande BW de 125 kHz et un Coding Rate de 4/5, est associé un numéro de débit (« DataRate » ou « DR ») allant de 0 (le plus faible débit) à 5 (le plus fort débit).

Les équipements radio qui utilisent les technologies de type LoRA sont avantageux en ce que ce sont généralement des équipements électroniques à faible consommation, de taille réduite et de faible puissance d'émission radio, et permettant de communiquer sur des longues distances.

Utilisant la technologie LoRa à étalement de spectre, un module radio LoRaWAN peut établir un lien radio bidirectionnel avec une ou plusieurs passerelles différentes. Un module radio LoRaWAN possède un identifiant unique.

Les passerelles LoRaWAN possèdent également toutes un identifiant unique et peuvent décoder de façon simultanée plusieurs canaux fréquentiels sur plusieurs facteurs d'étalement de spectre SF.

Le réseau de commande 3 représenté comprend une unité de commande 310, 311, 312 et, dans le sens passerelle vers unité de commande :
- un concentrateur réseau 32 ;
- un concentrateur applicatif 33 ; et
- une application ou serveur Web 34 implémentée dans l'unité de commande.

Les liaisons (ou liens) de communication réseau 30 peuvent être des liens TCP/IP (couche transport/couche réseau), optionnellement avec un protocole de sécurisation des échanges sur Internet (SSL, TLS typiquement) disposé entre la couche transport (TCP...) et la couche application (HTTP, FTP, SMTP, MQTT, ...).

A la réception d'une trame LoRaWAN provenant d'un module radio d'un dispositif de test, une passerelle transfère sur le lien IP (utilisant le protocole MQTT en TCP/IP le plus souvent), un paquet en y rajoutant des informations radio : on parle ici de lien « montant ». Ces informations sont à destination du concentrateur réseau 32. Dans l'autre sens, c'est-à-dire dans le lien dit « descendant », les informations sont transférées du concentrateur réseau 32 au module radio d'un dispositif de test. Chaque passerelle LoRaWAN a ainsi une fonction de « passe-plat » (« packet forwarder » en anglais) entre un lien radio utilisant la technologie LoRa et un lien IP, sans implémenter d'intelligence ou de filtrage particulier.

Le concentrateur réseau 32 (« network server » en anglais) a pour fonction principale de supprimer les doublons issus des informations montantes. En effet, puisqu'un paquet issu d'un équipement radio peut être réceptionné de façon simultanée par plusieurs passerelles, l'information se retrouve dupliquée. Pour la voix descendante, le concentrateur réseau sélectionne une seule passerelle pour émettre le paquet. Une clef d'authentification A (par exemple de type « NwkSKey ») peut être utilisée par le concentrateur réseau pour authentifier l'équipement radio.

Le concentrateur applicatif 33 a pour fonctions principales d'authentifier et de déchiffrer les informations montantes du concentrateur réseau ou les informations descendantes vers le concentrateur réseau. Le concentrateur applicatif peut, à titre subsidiaire, stocker des informations.

Le concentrateur applicatif peut disposer de clefs de déchiffrement C (par exemple de type « AppSKey ») partagées avec chacun des équipements radio des dispositifs de test.

Une autre fonction optionnelle du concentrateur applicatif est de décoder (respectivement encoder) la trame radio.

L'application ou serveur Web 34 est la dernière brique logicielle, en bout de chaîne, d'une architecture LoRaWAN standard. Dans le cas d'un dispositif de test de détecteur selon l'invention, l'application permet de réaliser l'encodage et le décodage, et de gérer les instructions de commande et les informations en provenance des dispositifs de test ou vers les dispositifs de test. Les interfaces de communication de l'application ou serveur Web utilisent généralement le protocole HTTP ou le protocole MQTT de sorte que l'interface graphique utilisateur est accessible depuis un navigateur Internet. Cette brique logicielle peut être aisément implémentée dans l'unité de commande.

Dans le système représenté en figure 6A, l'unité de commande 310 est représentée sous forme d'ordinateur portable, mais elle peut être un ordinateur de bureau, une tablette, un téléphone intelligent (ou Smartphone), et plus généralement n'importe quel dispositif « client » respectant les interfaces du serveur Web 34 (typiquement HTTP). L'unité de commande est ainsi capable de piloter à distance tout dispositif de test via son module radio. Dans ce cas, le réseau de commande 3 est un réseau IP et les liaisons de communication réseau 30 sont des liaisons IP filaires. La figure 6B montre le cheminement des données (représentées par des flèches) entre l'unité de commande 310 et le dispositif de test 101 via le concentrateur applicatif 33, le concentrateur réseau 32 et la passerelle 21. La référence F représente la fenêtre d'écoute LoRaWAN.

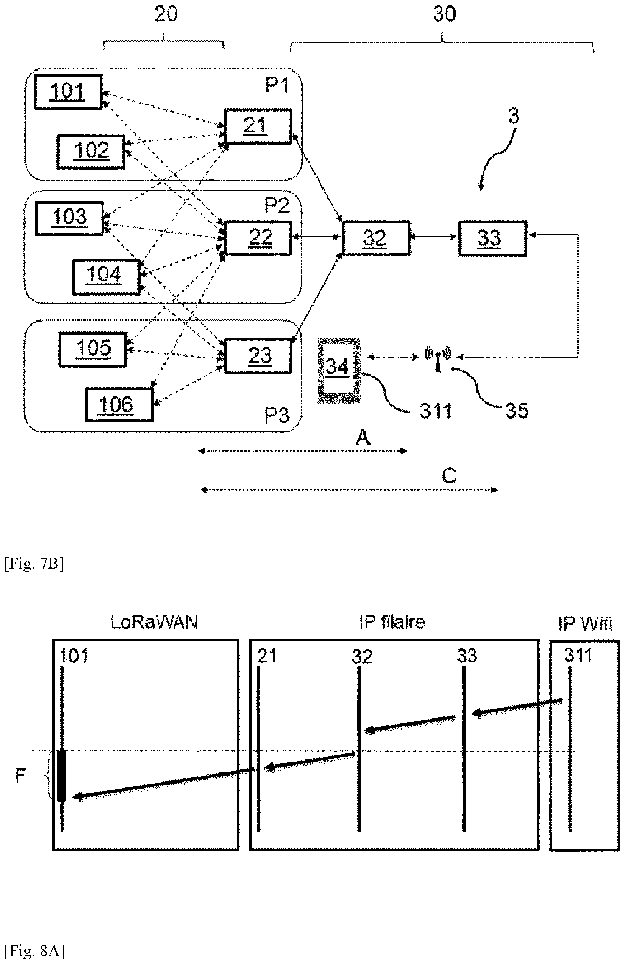

Dans le système représenté en figure 7A, l'unité de commande 311 est représentée sous forme d'une tablette ou d'un Smartphone. Dans ce cas, le réseau de commande 3 est un réseau IP et les liaisons de communication 30 comprennent des liaisons IP filaires entre les passerelles et le concentrateur applicatif 33 et entre le concentrateur applicatif 33 et une borne wifi 35, ainsi qu'une liaison sans fil entre la borne wifi et l'unité de commande 311. La figure 7B montre le cheminement des données (représentées par des flèches) entre l'unité de commande 311 et le dispositif de test 101 via le concentrateur applicatif 33, le concentrateur réseau 32 et la passerelle 21. La référence F représente la fenêtre d'écoute LoRaWAN.

Au lieu d'une borne Wifi, il peut s'agir d'une station cellulaire.

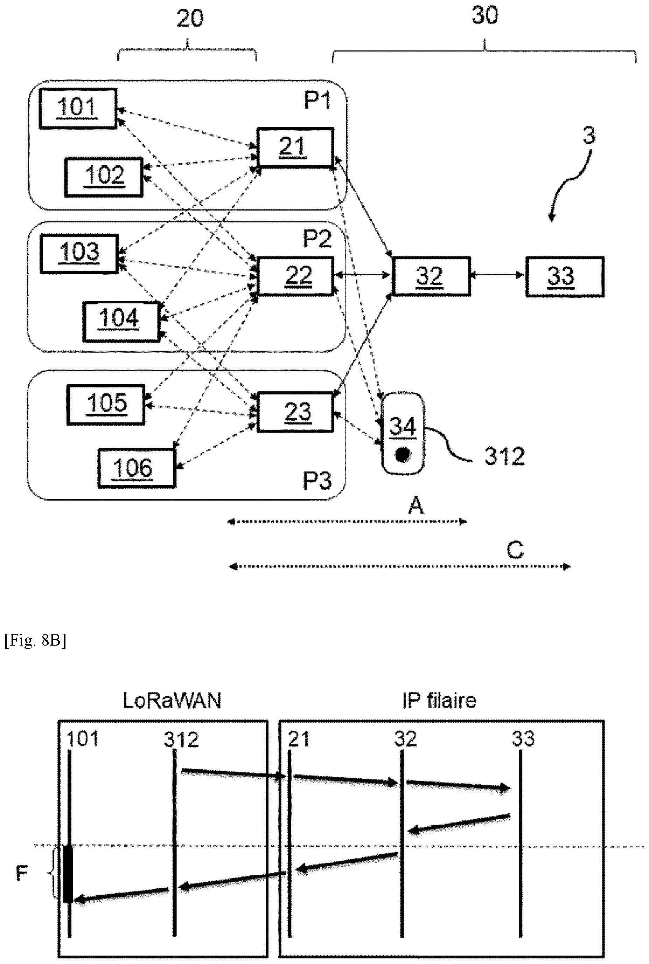

Dans le système représenté en figure 8A, l'unité de commande 312 est représentée sous forme d'une télécommande LoRaWAN, qui peut être plus généralement un dispositif portable équipé d'un composant radio compatible avec le protocole LoRaWAN. Dans ce cas, le réseau de commande 3 comprend des liaisons IP filaires entre les passerelles, le concentrateur réseau 32 et le concentrateur applicatif 33 et des liaisons radio LoRa entre les passerelles et la télécommande LoRaWAN. La figure 8B montre le cheminement des données (représentées par des flèches) entre l'unité de commande 312 et le dispositif de test 101 via la passerelle 21, le concentrateur réseau 32 et le concentrateur applicatif 33. La référence F représente la fenêtre d'écoute LoRaWAN.

Dans les trois configurations, le mécanisme de déclenchement du test suit une procédure Client(s) / Serveur. Avec une liaison radio LoRaWAN, le serveur est le concentrateur applicatif qui peut posséder deux interfaces et deux types de clients:
- une interface LoRaWAN à travers le concentrateur réseau, et le client est un dispositif radio dédié de type télécommande LoRaWAN ;
- une interface IP, par exemple de type HTTP(s), MQTT(s), CoAP(s), et le client est n'importe quel type de dispositif respectant les interfaces du serveur Web et ce, soit en filaire (par exemple via un câble RJ45), soit en sans-fil (Wifi, cellulaire...), soit en mixte.

Ainsi, une telle architecture LoRaWAN permet de réaliser sur commande l'activation d'un ou de plusieurs dispositifs de test de détecteur à distance, c'est-à-dire sans avoir à se rendre sur la zone testée. La commande peut être réalisée depuis un ordinateur de bureau, un ordinateur portable, une tablette et/ou un téléphone intelligent, et plus généralement, depuis n'importe quel dispositif « client » respectant les interfaces du serveur Web, ou encore par une télécommande LoRaWAN.

La bande de fréquence 868 MHz (pour l'Europe) est bien adaptée au domaine de l'invention car elle permet un bon compromis entre la zone de couverture radio et la capacité du système de test (nombre de dispositifs couverts par passerelle) pour couvrir un bâtiment.

Le protocole de communication LoRaWAN standard nécessite l'utilisation de classes (A, B et C). La principale différence entre ces classes réside dans le nombre de fenêtres allouées par l'équipement radio pour la réception des messages envoyés par le réseau de commande. Ainsi on différencie :
- La classe A qui permet l'émission d'un paquet d'informations suivi par deux fenêtres d'écoute ; uniques opportunités pour permettre la descente d'information depuis l'unité de commande vers l'équipement radio. Cette classe permet une grande autonomie de consommation électrique des dispositifs de test. L'inconvénient est que les dispositifs de test émettent de manière aléatoire (possibilité de collision de données) avec une durée d'écoute faible (peu de temps pour réagir au niveau du réseau de commande). La réactivité du réseau de commande est donc conditionnée par la périodicité d'émission des paquets issus des équipements radio. Cela a pour conséquence, à plus grande fréquence d'émission, une plus faible autonomie pour présenter une latence plus faible ;
- La classe B qui permet l'émission d'un paquet d'informations suivi par de multiples fenêtres d'écoute dont la périodicité est optimisée (par exemple toutes les minutes) suivant un compromis entre latence et autonomie ;
- La classe C qui permet l'émission d'un paquet d'informations suivi par au moins une fenêtre d'écoute continue permettant une latence quasi nulle (très grande réactivité des dispositifs de test, mais plus faible autonomie).

Les inventeurs proposent en première variante, une classe A « TDMA » (accès multiple à répartition dans le temps ou « time division multiple access » en anglais) qui permet d'éviter les risques de collisions de paquets radio sur un même canal de fréquence radio. Cela permet de s'affranchir d'un accès en classe A aléatoire et d'améliorer ainsi considérablement la qualité de service. Le principe est une attribution temporelle synchronisée et fréquentielle des instants d'émission pour maîtriser ces collisions. Dans cette variante, le compromis entre latence et consommation est optimisé en fonction de la latence maximale demandée par l'opérateur et d'un planning de test préétabli à l'aide d'un algorithme dédié.

En outre, afin d'optimiser l'autonomie du dispositif de test, les inventeurs ont proposé un protocole de communication basé sur les spécifications LoRaWAN standard en développant quatre états de fonctionnement des dispositifs de test :
- Un état dit « relâché » où le dispositif de test est en veille profonde (pas d'émission, pas d'écoute) pendant une période fixée par l'unité de commande (par exemple 6 mois). De préférence, un état « relâché » ne peut passer qu'en état « transitoire ». Cela améliore la sécurité du système de test d'une part et son autonomie d'autre part ;
- Un état dit « transitoire » où le dispositif de test est, de préférence pendant une durée maximale de quelques jours (par exemple 5 jours), en classe A avec une périodicité de plusieurs minutes à plusieurs heures. L'état transitoire offre une qualité de service potentiellement faible, la qualité dépendant du nombre de dispositifs de test, et l'accès au médium de communication (air par ondes radio) étant purement aléatoire et le temps de réactivité, défini comme le temps écoulé entre la demande issue de l'unité de commande et l'actionnement d'un dispositif de test, peut ne pas être compatible avec l'attente de l'opérateur lors des tests, mais il a pour fonction de permettre à l'opérateur de programmer la date et l'heure des tests basés sur son planning. Cette programmation se fait en commutant l'équipement radio dans un mode ayant une réactivité plus faible (mode soutenu). Une procédure de synchronisation entre le temps absolu du serveur du réseau de commande et les horloges des dispositifs radio est réalisée et confirmée par un message d'acquittement. Un état transitoire peut passer ensuite en état « relâché » ou en état « soutenu ». Dans cet état transitoire, la périodicité d'émission des paquets radio issus des dispositifs de test est aléatoire. Le risque de collision est non contrôlé et la qualité de service diminue avec le nombre de dispositifs de test et la périodicité d'émission de ces derniers ;
- Un état dit « soutenu » où le dispositif est soit en écoute permanente (classe C), soit en écoute avec un temps de latence compatible avec les besoins de l'opérateur, soit généralement de l'ordre de la minute (classe A « TDMA »). Cet état ne doit durer de préférence que quelques heures (par exemple 4 heures) du fait de la consommation électrique maximale et possède une grande qualité de service (réactivité maximale de l'ordre de la minute). Cet état permet à tout moment de passer à l'état « test » ou de passer à l'état « transitoire » si aucun test n'est réalisé à l'issue de la période d'état « soutenu », et il permet de proposer à l'opérateur une réactivité maximale entre la demande de test issue de l'unité de commande et l'actionnement réel du test sur le dispositif de test. Dans cet état soutenu, la périodicité d'émission des paquets radio issus des dispositifs de test est synchronisée avec une horloge unique dans tout le réseau (réseau radio local et réseau de commande). L'attribution des fenêtres temporelles et fréquentielles est optimisée par un algorithme qui limite le risque de collision ;
- Un état dit « test » où le dispositif de test actionne la cartouche de gaz suite à une commande lancée par l'unité de commande. Cet état dure au maximum 1 minute. Il passe ensuite directement en état « transitoire » dans l'attente de reprogrammation de la date du prochain cycle.

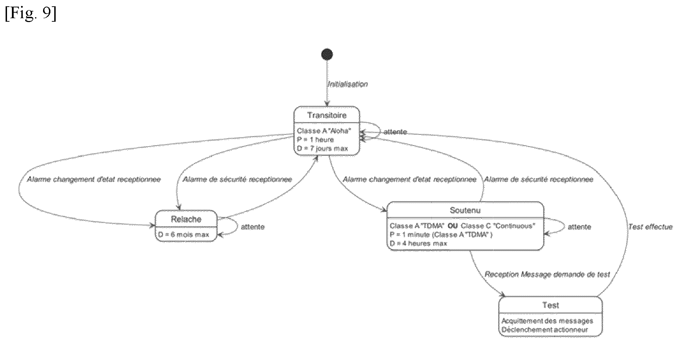

Un exemple de graphe d'état donné en figure 9 illustre ce protocole de fonctionnement.

Ce protocole permet d'avoir un lien bidirectionnel.

Ce protocole développé consiste en une couche logicielle adossée sur la couche LoRaWAN de l'équipement radio du dispositif de test et sur le concentrateur applicatif.

Alternativement à la technologie radio LoRa autour de la fréquence de 868 MHz, il est possible d'employer une autre bande de fréquence, par exemple la fréquence de 915 MHz (pour les USA), ou de 780 MHz (pour l'Asie), ou encore l'une des fréquences suivantes : 169 MHz, 433 MHz, 2,4 GHz, 5,8 GHz ou tout autre bande de fréquence adaptée située entre 9 kHz et 300 GHz. Alternativement à la technologie radio LoRa, le réseau radio peut être conforme à une autre technologie radio faisant partie des réseaux étendus à faible consommation énergétique, définis par le terme anglais « LPWAN » pour « Low Power Wide Area Network ».

Encore alternativement, le réseau radio peut être conforme à d'autres technologies telles que la norme Zigbee (IEEE 802.15.4), le Bluetooth^{™}, le Bluetooth^{™} Low Energy qui sont des réseaux personnels sans fil (« WPAN » pour « Wireless Personal Area Network » en anglais).

L'homme du métier saura adapter le réseau de commande en fonction de la technologie radio adoptée, en fonction du type d'unité de commande souhaitée (ordinateur de bureau, ordinateur portable, une tablette, un téléphone intelligent, superviseur, télécommande radio ...) et en fonction des interfaces et des supports de communication souhaités dans le réseau de commande.

Le système de test selon l'invention peut s'intégrer aisément à un système existant de détection. Par exemple, un système existant de détection est composé de détecteurs incendie dont les informations remontent en filaire à un système de supervision, généralement via un équipement de contrôle et/ou de signalisation qui communique avec des moyens de mise en sécurité, par exemple pour activer des ventilateurs de désenfumage et/ou permettre l'évacuation des personnes au moyen de diffuseurs sonore ou lumineux. Le système de test selon l'invention peut communiquer avec le système de supervision au moyen de la (ou des) passerelle(s), et tous les éléments du système existant de détection peuvent être conservés et fonctionner de la même manière. L'unité de commande du système de test peut être comprise dans ou être associée avec le système de supervision (par exemple l'application peut être intégrée au niveau du système de supervision pour piloter à distance les différents dispositifs de test).

Sauf indication contraire ou évidente, les différents modes et variantes de réalisation présentés peuvent être combinés entre eux.

En outre, la présente invention n'est pas limitée aux modes de réalisation précédemment décrits mais s'étend à tout mode de réalisation entrant dans la portée des revendications.

## Revendications

1. Dispositif de test (1) pour un détecteur (51, 52, 53) basé sur la détection de gaz ou d'un mélange gaz/particules, le dispositif comprenant :
- un moyen de solidarisation (11, 110, 115) adapté pour solidariser le dispositif de test au détecteur à tester ;
- un contenant (13) configuré pour recevoir au moins un gaz de test, ou au moins un mélange gaz/particules de test ;
- un moyen de diffusion (12) associé au contenant (13) et adapté pour diffuser le gaz de test ou le mélange gaz/particules de test dans le détecteur ;
- une source d'énergie (160), par exemple un moyen de stockage d'énergie, adapté pour rendre le dispositif de test autonome en énergie ; et
- un équipement radio (140) apte à gérer une communication radio locale, de préférence une communication radio bidirectionnelle, pour au moins déclencher à distance, depuis un moyen de commande, la diffusion du gaz test ou le mélange gaz/particules de test dans le moyen de diffusion (12), et ainsi dans le détecteur.

2. Dispositif de test (1) selon la revendication 1, le contenant (13) étant apte à recevoir ou étant une cartouche de gaz test ou de mélange gaz particules test.

3. Dispositif de test (1) selon l'une des revendications 1 ou 2, le moyen de diffusion (12) comprenant un diffuseur (121) configuré pour acheminer le gaz de test ou le mélange gaz/particules de test au plus près de l'entrée de gaz du détecteur (51, 52, 53).

4. Dispositif de test (1) selon l'une des revendications 1 à 3, le moyen de diffusion (12) comprenant un élément (122) apte à coopérer avec le moyen de solidarisation (11).

5. Dispositif de test (1) selon l'une des revendications 1 à 4, comprenant en outre un actionneur (150) pilotable à distance et apte à actionner la diffusion du gaz de test ou du mélange gaz/particules de test dans le moyen de diffusion (12), et ainsi dans le détecteur.

6. Dispositif de test (1) l'une quelconque des revendications 1 à 5, le moyen de solidarisation (11) comprenant au moins un doigt d'accrochage (111, 112, 113) dont une première extrémité (111A, 112A, 113A) présente une forme en crochet apte à coopérer avec un renfoncement dans le détecteur.

7. Dispositif de test (1) l'une quelconque des revendications 1 à 5, le moyen de solidarisation (110, 115) comprenant une bride de fixation (110B, 115) apte à enserrer le détecteur.

8. Dispositif de test (1) selon la revendication 7, le moyen de solidarisation (110) comprenant en outre au moins une extrémité (110C, 110D) adaptée pour régler le serrage ou le desserrage de la bride de de fixation (110B) autour du détecteur, ladite au moins une extrémité étant de préférence positionnée dans l'axe du centre de gravité du dispositif de test.

9. Dispositif de test (1) selon l'une quelconque des revendications précédentes, le moyen de solidarisation (110) comprenant en outre un moyen de réglage (110A) de la position relative du dispositif de test par rapport au détecteur.

10. Dispositif de test (1) selon l'une quelconque des revendications précédentes, comprenant une base de temps précise, par exemple une horloge en temps réel, et un moyen de mise en veille et de réveil périodique du dispositif de test, la périodicité de réveil étant adaptable.

11. Dispositif de test (1) selon l'une quelconque des revendications précédentes, la communication radio locale étant conforme à la technologie LoRa, l'équipement radio (140) comprenant un composant compatible avec la technologie LoRa, avec un protocole de communication basé sur le protocole LoRaWAN.

12. Dispositif de test (1) selon la revendication 11 en combinaison avec la revendication 10, le moyen de mise en veille et de réveil périodique du dispositif de test (1) étant intégré dans une couche logicielle adossée sur la couche LoRaWAN de l'équipement radio (140) du dispositif de test (1).

13. Système de test pour au moins un détecteur (51, 52, 53) basé sur la détection de gaz ou d'un mélange gaz particules, ledit système de test comprenant au moins un dispositif de test (1, 101, 102, 103, 104, 105, 106) choisi selon l'une quelconque des revendications 1 à 12 et comprenant en outre :
- au moins une passerelle radio (2, 21, 22, 23), et
- un réseau de commande (3) comportant une unité de commande (31, 310, 311, 312) apte à commander à distance le au moins un dispositif de test et au moins une liaison de communication réseau (30) entre ladite unité de commande et ladite au moins une passerelle radio ;
la au moins une passerelle radio étant apte à relier l'équipement radio (140) du dispositif de test et le réseau de commande (3).

14. Système de test selon la revendication 13, comprenant un dispositif de test selon l'une des revendications 10 ou 12, le réseau de commande (3) comprenant un serveur de diffusion de temps universel apte se synchroniser avec la base de temps du dispositif de test, l'unité de commande (31, 310, 311, 312) étant apte à donner des instructions de mise en veille et de réveil au dispositif de test.

15. Système de test selon l'une des revendications 13 ou 14, le réseau de commande (30) étant un réseau IP, l'unité de commande (310, 311) comprenant une couche IP, l'unité de commande (310, 311) étant par exemple un ordinateur de bureau, un ordinateur portable, une tablette, un téléphone intelligent.

16. Système de test selon l'une des revendications 13 à 15, la au moins une liaison de communication réseau (30) étant entièrement filaire.

17. Système de test selon l'une des revendications 13 à 16, la communication radio locale étant conforme à la technologie LoRa, l'équipement radio (140) du dispositif de test comprenant un composant compatible avec la technologie LoRa, avec un protocole de communication basé sur le protocole LoRaWAN et la au moins une passerelle radio (2, 21, 22, 23) étant une passerelle LoRaWAN.

18. Système de test selon la revendication 17, le réseau de commande (3) comprenant en outre un concentrateur réseau (32) et un concentrateur applicatif (33).

19. Système de test selon la revendication 18 en combinaison avec la revendication 14, le moyen de mise en veille et de réveil périodique du dispositif de test étant intégré dans une couche logicielle adossée sur la couche LoRaWAN de l'équipement radio (140) dudit dispositif de test et sur le concentrateur applicatif (33).

20. Système de test selon l'une des revendications 18 ou 19, le réseau de commande (3) comprenant plusieurs liaisons de communication (30) comportant une partie filaire reliant la au moins une passerelle (2, 21, 22, 23), le concentrateur réseau (32) et le concentrateur applicatif (33), et une partie sans-fil, par exemple en Wifi, reliant le concentrateur applicatif (33) et l'unité de commande (311), les liaisons de communication pouvant être des liaisons de communication IP.

21. Système de test selon l'une des revendications 18 ou 19, le réseau de commande (3) comprenant plusieurs liaisons de communication comportant au moins une liaison de communication IP, filaire et/ou sans-fil, entre la au moins une passerelle (2, 21, 22, 23), le concentrateur réseau (32) et le concentrateur applicatif (33) et au moins une liaison de communication radio LoRa entre la au moins une passerelle et l'unité de commande (312), l'unité de commande (312) comprenant un dispositif portable équipé d'un composant radio compatible avec la technologie LoRa.

22. Système de détection comprenant un système de test selon l'une des revendications 13 à 21 et un détecteur basé sur la détection de gaz ou d'un mélange gaz particules, par exemple un détecteur incendie basé sur la détection de fumée.
